# EUROPEAN PATENT SPECIFICATION

(11) **EP 2 574 333 B1**
(45) Date of publication and mention of the grant of the patent: **11.01.2017**
(21) Application number: 11182999.0
(22) Date of filing: 27.09.2011
(51) Int. Cl.: A61K 9/00, A61K 31/198, A61K 9/46, A61K 9/20, A61K 31/7072, A61P 11/00, A61P 1/16

(54) **N-acetylcysteine effervescent tablet and its therapeutical applications**
N-Acetylsystein-Brausetablette und ihre therapeutischen Anwendungen
Comprimé effervescent de N-acétylcystéine et ses applications thérapeutiques

(43) Date of publication of application: 03.04.2013
(73) Proprietor: Friulchem SpA, 33099 Vivaro (IT)
(72) Inventor: PERRI, Lidia, 20052 MONZA (IT); COPPI, Germano, 27028 SAN MARTINO SICCOMARIO (IT)
(74) Representative: Regimbeau

(56) References cited:
- WO-A1-2010/090612

## Description

The present invention is related to pharmaceutical composition comprising N-acetylcysteine (NAC) in the form of an effervescent tablet having no sulfur smell and no sulfur taste.

N-acetylcysteine (NAC) is an acetylated derivative of the amino acid L-cysteine which has a free thiol group. NAC was described in patent US 3,184,505.

NAC is indicated as adjuvant therapy (NAC is used in combination with antibiotics or other drugs) for patients with abnormal, viscid, or inspissated mucous secretions in such conditions as chronic respiratory disorders, acute bronchopulmonary disease, tracheostomy care, during anesthesia, atelectasis due to mucous obstruction, and diagnostic bronchial studies. Oral NAC is a very effective mucolytic agent and is useful in decreasing the number of exacerbations in patients with chronic bronchitis. When given in combination with cefuroxime, NAC increases the antibiotic's penetration into bronchial secretion. (Drugdex Drug Evaluations, 2011).

N-acetylcysteine (NAC) is also indicated as an antidote to prevent or lessen hepatic injury which may occur after ingestion of potentially hepatotoxic quantity of acetaminophen. NAC is considered the drug of choice for prevention against liver damage in severe acetaminophen intoxication. In one analysis, 11,195 cases of suspected acetaminophen overdose were reviewed. It was concluded that NAC treatment should be started within eight hours of overdose but it is still indicated at least as late as 24 hours after ingestion. It was also concluded that a 72-hour regimen of oral N-acetylcysteine is as effective as the 24-hour regimen and may be superior when treatment is delayed (Drugdex Drug Evaluations, 2011).

N-acetylcysteine is also indicated in many other situations, like :
1. In a randomized, 2-center, double blind study in patients with chronic kidney disease, volume supplementation by sodium bicarbonate plus NAC was superior to the combination of normal saline with NAC alone or with the addition of ascorbic acid in preventing contrast agent-induced nephrotoxicity (CIN) in patients at medium to high risk. (Drugdex Drug Evaluation, 2011).
2. Other potential uses of NAC include protection against oxidant (free radicals) damage and as protection against cytotoxic agents such as ifosfamide and cyclophosphamide. (Drugdex Drug Evaluation, 2011).
3. Glutathione depletion has been implicated in the pathology of a number of diseases including infection by immunodeficiency virus (HIV). In HIV infection, cysteine/glutathione depletion is known to impair T-cell function and is associated with impaired survival of subjects with less than 200 CD4 T-cells per µl. Thus pharmaceutical compounds, like N-acetylcysteine, that replenish or elevated glutathione levels, work, at least in part, through enhancement of the defense mechanism seemingly utilized to normally protect tissue from reactive oxidative intermediates (ROI) mediate damage.
4. Nucleoside reverse transcriptase inhibitors (NRTIs), like for example azidothymidine (AZT, zidovudine), are often given in combination therapies with other anti-retroviral drugs to treat HIV. Long term therapy with AZT is commonly associated with dose-dependent hematologic toxicity which shows low erythrocyte counts and elevated mean red cell volume, and with muscle fiber toxicity. Some studies indicate that AZT's toxic interactions result from generation of reactive oxygen species (ROI) that react and deplete intracellular glutathione levels. Also in this case the treatment with N-acetylcysteine can be very useful.

Regarding the safety of NAC, this drug is very well tolerated (RTECS, 2010) (Fluimucil ® 600 mg leaflet). The Toxic Dose Low (TDLO) in human after intravenous route is 150-200 mg/kg. The TDLO in rats after intraperitoneal route is 150-200 mg/kg. The TDLO in mouse after intraperitoneal route is 408-450 mg/kg. The TDLO in mouse after oral route is 300 mg/kg.

The data obtained from the chronic toxicity in rats revealed a no observed effect level (NOEL) dose of about 1000 mg/kg/day for 12 months (Johnston et al., Seminars in Oncology, 1983, 10 Suppl,: 17-24).

NAC is currently commercialized under the trademark Fluimucil®. In Fluimucil® effervescent tablets, 600 mg of active ingredient NAC is mixed with 680 mg citric acid, 573 mg sodium hydrogen carbonate, 200 mg lemon flavouring, and 20 mg aspartame.

The Fluimucil® effervescent tablets comprise important amounts of lemon flavour to mask the sulfuric taste which nevertheless is present. In addition, after dissolution the Fluimucil® effervescent tablets have an unpleasant sulfuric smell and a marked lemon taste.

NAC is rapidly absorbed after oral administration in both animals and humans. The maximum plasma concentration is reached 2-3 hours after administration and the plasma half-life is 6.3 hours. NAC undergoes extensive hepatic metabolism, resulting in low bioavailability of about 10% for the unchanged molecule. As expected, NAC cannot be detected in plasma or bronchoalveolar lavage fluid following oral administration for 4-14 days. In contrast, cysteine and GSH levels increased transiently in plasma and lung after administration of NAC 600 mg once daily. In patients with chronic obstructive pulmonary disease (COPD), however, plasma concentration of Glutathione (GSH) were unchanged after this dose of NAC, whereas 600 mg three times daily increased plasma GSH levels.

The aim of the invention is to propose tablets comprising 1200 mg of NAC. This dose is expressed in mg/kg equal to 1200: 60 kg = 20 mg/kg which is 50 times less referred to NOEL in rats always for oral route. This value is also about 10 times less referred to TDLO in human after intravenous route. Consequently, the 1200 mg oral dose of NAC will not cause toxic effects in humans.

WO2010/090611 discloses compositions comprising 900-2500 mg of NAC. In particular WO2010/090611 discloses compositions comprising: 1200 mg NAC, 60 mg sodium chloride, 1035 mg citric acid anhydride, 770 mg sodium hydrogen carbonate, 30 mg aspartame, 20 mg lemon flavor, 25 mg PEG 6000, and 60 mg PVP K-30. Sodium chloride is added as taste regulator. The process for manufacturing the tablets is not disclosed.

WO2010/090612 relates to taste and odor masked pharmaceutical compositions in the from of effervescent tablets for the treatment of chronic respiratory diseases and for the prevention of liver failure, said composition comprising 1200mg NAC (N-Acetylcystein) in the presence of sodium chloride for regulating the taste.

However it remains need of pharmaceutical compositions containing NAC which do not have sulfur smell and/or sulfur taste and which does not comprise sodium chloride, which may be contraindicated in some patients.

The compositions of the invention have no sulfur smell and/or sulfur taste. In the present invention, the pharmaceutical composition in the form of an effervescent tablet comprises:
(a) 1200 mg of N-acetylcysteine; and
(b) at least 1200 mg of sodium hydrogen carbonate; and
(c) less than 1200 mg of anhydrous citric acid; and
(d) at least one lubricant and/or glidant agent; and
(e) at least one sweetener and/or flavouring agent; and
said pharmaceutical composition having no sulfur smell and taste as well as a pH in the range of 4.1 and 4.5 after tablet dissolution.

The composition of the invention preferably contains NAC as the active ingredient in an amount ranging from 30 to 35% by weight, on the basis of the total weight of the composition.

Sodium hydrogen carbonate and anhydrous citric acid are used as disintegrant and effervescent agents.

Sodium hydrogen carbonate is preferably present in the composition in an amount ranging from 30 to 40% by weight, on the basis of the total weight of the composition. In a preferred embodiment, in the pharmaceutical composition of the invention the amount of sodium hydrogen carbonate is between 1250 mg and 1350 mg, preferably between 1250 mg and 1300 mg, more preferably equal to 1313 mg.

Anhydrous citric acid carbonate is preferably present in the composition in an amount ranging from 20 to 30% by weight, more preferably 25 to 30% by weight, on the basis of the total weight of the composition. In a preferred embodiment, in the pharmaceutical composition of the invention the amount of anhydrous citric acid is between 1050 mg and 1100 mg, preferably equal to 1087 mg.

In a preferred embodiment, the lubricant and/or glidant agent is selected from the group consisting of magnesium stearate, sodium stearyl fumarate, sodium benzoate, polyethylene glycol with molecular weights of 6000 and above, and colloidal silicon dioxide. More preferably, the lubricant and/or glidant agent is a lubricant agent, in particular a polyethylene glycol with molecular weights of 6000 and above, preferably polyethylene glycol 6000.

The lubricant and/or glidant agent is preferably present in the composition in an amount ranging from 0.2 to 2% by weight, more preferably 0.4 to 1% by weight, on the basis of the total weight of the composition In the pharmaceutical composition of the invention the amount of lubricant and/or glidant agent is preferably between 5 mg and 60 mg, more preferably between 10 mg and 50 mg, even more preferably equal to 30 mg.

When the lubricant and/or glidant agent is a mixture of a lubricant agent and a glidant agent, the masse ratio lubricant:glidant is preferably comprised between 70:30 and 90:10, more preferably between 80:20 and 85:15.

In a preferred embodiment, the sweeteners are selected from the group consisting of sucralose, sucrose, fructose, glucose, galactose, xylose, dextrose, levulose, lactose, maltose, maltodextrin, mannitol, maltitol, maltol, sorbitol, xylitol, erythritol, lactitol, isomalt, corn syrup, saccharin, saccharin salts, acesulfame potassium, aspartame, D-tryptophan, monoammonium glycyrrhizinate, neohesperidin dihydrochalcone, thaumatin, nicotame, alitame, stevioside and cyclamate. More preferably, the sweetener is aspartame.

The sweetener is preferably present in the composition in an amount ranging from 0.2 to 2% by weight, more preferably 0.3 to 0.8% by weight, on the basis of the total weight of the composition In the pharmaceutical composition of the invention the amount of sweetener is preferably between 5 mg and 30 mg, more preferably between 15 mg and 25 mg. In a preferred embodiment, the composition contains 20 mg of aspartame.

In a preferred embodiment, the flavouring agents are selected from the group consisting of natural aroma oils (e.g. peppermint oil, Partridge currant oil, clove bud oil, parsley oil, eucalyptus oil, lemon oil, orange oil, etc.), menthol, mint, anethole, methylsalicylate, eucalyptol, cinnamon, 1-methyl acetate, salvia, eugenol, oxanone, alpha-ionone marjoram, lemon, orange, propenyl guaethol, cinnamon, vanilla, thymol linalool cinnamaldehyde glycerol acetal, N-substitutedp-menthane- 3-carboxyamide and 3,1- methoxy propane 1,2-diol. More preferably, the flavouring agent is lemon oil.

The flavouring agent is preferably present in the composition in an amount ranging from 2 to 10% by weight, more preferably 3 to 8% by weight, on the basis of the total weight of the composition. In the pharmaceutical composition of the invention the amount of flavouring agent is preferably between 100 mg and 300 mg, more preferably between 150 mg and 250 mg. In a preferred embodiment, the composition contains 200 mg of lemon oil.

The composition of the invention preferably contains solely the components quoted in points (a), (b), (c), (d) and (e) above. In particular, the composition of the invention does not comprise a sulphur taste regulator, such as sodium chloride, other than masking agent (sweetener and/or flavouring agent). Indeed, it has been surprisingly discovered that the formulation of the invention has no sulfur smell and no sulphur taste although no taste regulator is added. In particular, it has been surprisingly discovered that the formulation of the invention has no sulfur smell and no sulphur taste although no taste regulator is added and the quantity of masking agent (sweetener and/or flavouring agent) is less than the double of the quantity of masking agent in Fluimucil® effervescent tablets.

In the most preferred embodiment, the pharmaceutical composition contains solely: 1200 mg ofNAC, 1313 mg of sodium hydrogen carbonate, 1087 mg of anhydrous citric acid, 30 mg of PEG 6000, 20 mg of aspartame and 200 mg of lemon oil. The pH of this formulation after tablet dissolution is of 4.3

The disintegration time of the effervescent tablets according to the invention in water is less than 3 min, preferably less than 2 min.

The manufacturing of effervescent tablets comprising 1200 mg of NAC presents technical difficulties notably due to the high weight and dimension of tablets and to the bad process-ability of NAC. In order to solve the above problems, manufacturing process of effervescent tablet is proposed in the present application.

The present invention is also directed to a process for preparing a pharmaceutical composition according to the invention, which comprises
- (1) the wet granulation of N-acetylcysteine with anhydrous citric acid and optionally sodium hydrogen carbonate, then
- (2) the mixing of the granulate obtained after step (1) with the remaining excipients and
- (3) the compression of the tablet from the formulation obtained after step (2).

We will now explain the process in more details, according to the best embodiment.

In step (1), N-acetylcysteine, anhydrous citric acid and optionally sodium hydrogen carbonate are mixed under dry conditions. Powders are mixed during sufficient time, at the appropriate speed, to obtain a blend visually homogeneous before starting granulation.

The amount of NAC is calculated is such a way that final tablets comprise 1200 mg ofNAC.

The amount of anhydrous citric acid is calculated is such a way that final tablets comprise less than 1200 mg, preferably between 1050 mg and 1100 mg, more preferably 1087 mg, of anhydrous citric acid; taking however in consideration that a part of the anhydrous citric acid can be provided by the granulation solution used in the following steps of the process.

In one advantageous embodiment of the invention, the amount of the anhydrous citric acid in the mix is of at least 80% by weight, compared to the total weight of anhydrous citric acid, preferably from 80% to 98% by weight, more preferably from 85% to 98% by weight, even more preferably from 90 % to 98% by weight, compared to the total weight of anhydrous citric acid.

Then the blend is submitted to the granulation process. The granulation solution is a liquid composition comprising water and eventually the remaining part of anhydrous citric acid.

In a preferred embodiment, the liquid composition comprises water and anhydrous citric acid. The mass ratio water:anhydrous citric acid preferably ranges from 70:30 to 30:70, more preferably from 50:50 to 30:70, even more preferably from 50:50 to 40:60.

The agitation is stopped when the granules show the required appearance, easily determined by the skilled person.

The granules are the dried as a temperature preferably ranging from 30°C to 70°C, more preferably from 40°C to 60°C. During the drying phase, the temperature of the product shall preferably not exceed 45°C. The drying conditions (temperature, duration) are such that the residual humidity of the granules is less than 0.2%, preferably less than 0.15 %. Then, granules are cooled down until the temperature of the product is preferably lower than 28°C.

The granules obtained after the above steps are screened, preferably through a sieve of 1.25 mm mesh size.

In industrial process, one can contemplate to perform the granulation process in at least 2 time; meaning that before performing the granulation, the granulation solution is divided into 2 parts for the 2 granulations (or into 3 parts for the 3 granulations, etc.).

After the screening step, all the granules are put together in a same tank.

Raw materials of the external phase (sodium hydrogen carbonate, lubricant and/or glidant agent, sweetener and/or flavouring agent), in amounts appropriate to obtain the desired quantities in the final tablet, are screened, preferably through a sieve of 1.25 mm. The screened materials are then poured into the tank containing all the granules.

Then, all the materials are mixed.

Tablets are pressed from the resulting final mixing. An external lubrication, with magnesium stearate for example, can be used to avoid sticking during the tableting operation. The resulting tablets are then packaged.

The disintegration time of the effervescent tablets obtained by the process of the invention in water is less than 3 min, preferably less than 2 min. The friability of the effervescent tablets obtained by the process of the invention in water is less than 2%, preferably less than 1%. The hardness of the tablets is between 14 and 18 Kp.

The tablets are preferably prepared in a controlled facility with controlled temperature and low relative humidity (20°C ± 1°C; RH 15% ± 5%).

The tablets of the invention can be used in many therapies and in many problems where toxicity due to ROI activity is present.

The invention is directed to tablets of the invention for their use in the treatment of abnormal, viscid, or inspissated mucous secretions. In particular the secretions may be the results of: chronic respiratory disorders, acute bronchopulmonary disease, tracheostomy care, during anesthesia, atelectasis due to mucous obstruction, and diagnostic bronchial studies.

The tablets of the invention can be used in combination with antibiotics, such as cephalosporin antibiotic in particular cefuroxime.

The invention is directed to tablets of the invention for their use in the treatment of hepatic injury after ingestion of hepatotoxic quantity of acetaminophen. NAC is considered the drug of choice for prevention against liver damage in severe acetaminophen intoxication.

The invention is directed to tablets of the invention for their use in the long term treatment of HIV with AZT. NAC will reduce the toxic effects linked to the use of

### AZT.

The invention is directed to tablets of the invention for their use in the prevention of oxidant damages and/or in the protection against cytotoxic agents such as ifosfamide and cyclophosphamide.

The tablets can be administered up to three times per day.

The following examples are put forth so as to provide those ordinary skill in the art with a complete disclosure and description of how to make and use the present invention, and are not intended to limit the scope of what the inventors regard as their invention nor they intended to represent that the experiments below all or the only experiments performed.

### Example 1

A patient having a fever can be treated with acetaminophen with a formulation comprising 15 mg/kg acetaminophen and N-acetylcysteine effervescent tablet (1200 mg) administered two or three times a day. For a child the dosages are half-tablet two or three times a day.

### Example 2

A patient having liver failure or liver damage or having elevated liver enzymes and fever who is already compromised can be treated with a cold, pain, antipyretic or other formulation; N-acetylcysteine effervescent tablet is to administered two or three times day. For a child the dosages are half-tablet two or three times a day.

### Example 3

A patient having HIV infection can be treated with a formulation comprising a therapeutically effective amount of AZT as a part of a multi-drug anti-viral regimen and a toxicity-reducing amount of N-acetylcysteine effervescent tablet administered two or three times day. For a child the dosages are half-tablet two or three times a day.

### Example 4

AZT and N-acetylcysteine can be administered perinatally and neonatally to prevent vertical transmission of the HIV virus to the child. Also in this case the N-acetylcysteine was administered to the mother as effervescent tablet two or three times per day.

### Example 5

A patient with abnormal, viscid or inspissated mucous secretion in chronic respiratory disorder, acute pulmonary disease, tracheotomy care, during anesthesia, atelectasis due to mucous obstruction and diagnostic bronchial studies has to be treated with N-acetylcysteine effervescent tablets two or three times daily under direction of a physician. For a child the dosages are half-tablet two or three times a day.

### Examples 6

In adults with chronic kidney disease N-acetylcysteine effervescent tablet two or three times daily is useful to prevent contrast-agent induced nephrotoxicity (CIN) in patients at medium to high risk.

### Example 7

In adult treated with cytotoxic agents such as ifosfamide and cyclophosphamide, N-acetylcysteine as effervescent tablet is administered two or three times daily to include protection against oxidant (free radicals) damage.

### Example 8 - manufacturing process

The effervescent tablets of the inventions are manufactured by the following process.

Residual humidity and temperature of the room are controlled during each step except for the weighing and the mixing for the preparation of N-acetylcysteine effervescent tablet.

### Step 0: raw materials weigh-in.

Raw materials are weighed in the weighing station.

### Step 1: preparation of the solution for granulation.

3.940 kg of purified water are weighed and put under agitation. Then, 5.440 kg of anhydrous citric acid are poured into the water and dissolved in thank with the agitation. The solution is conforming for the granulation step when it is limpid.

Once it is limpid, the solution is divided into 2 parts (for the 2 granulations).

### Step 2 a: dry mixing.

5.440 kg N-acetylcysteine and 51.630 kg anhydrous citric acid are introduced in a granulating blender. Powders are mixed during 10 minutes at 125 rotations per minute to obtain a blend visually homogeneous before starting granulation.

### Step 2 b : granulation.

The blend is put under agitation and the solution for granulation is poured into a granulating blender through a funnel. Then the granulation process is started. The agitation is stopped when the appearance of the granules is good. The duration of the granulation step 2 b was 6 minutes.

### Step 2 c: drying - cooling.

Granulate is quickly dried in the fluid bed dryer at the temperature of 50°C. Residual humidity was ≤ 0.15 %. During the drying phase, the temperature of the product did not exceed 45°C. Then, granule is cooled down in the same equipment at 5°C until the temperature of the product is lower than 28°C. The relative humidity of the granule is less than 0.15%.

### Step 3 : granules screening.

Granules (2 parts) are screened through a sieve of 1.25 mm mesh size and poured into the same 900L tank.

### Step 4 : external phase screening.

Raw materials of the external phase (131.300 kg sodium hydrogen carbonate, 20.000 kg lemon flavour, 2.000 kg aspartame and 3.000 kg PEG 6000) are screened through a sieve of 1.25 mm mesh size and poured into the tank containing the 2 parts of granules.

### Step 5 : Final mixing

A final mixing is performed with all raw materials in the tank at the speed of 10 rpm. The duration was 10 minutes. The residual humidity is less than 0.25%.

### Step 6: tabletting operation with an external lubrication.

Tablets are pressed from the final mixing. An external lubrication with magnesium stearate is used to avoid sticking during the tabletting operation.

Rotary press Courtoy R190FT or Fette 2200i or similar equipments were used.

The average mass of the tablet is 3850 mg. The uniformity of the mas was controlled and good.

The diameter of the tablet is 23 mm. The thickness is 3.10 to 3.30 mm. The hardness is 16 Kp The friability is less than 1%.

The disintegration time is less than 3 minutes.

The tablets do not smell sulphur. After dissolution into water, the limpid solution does not smell sulphur or taste sulphur. The limpid solution does not have a salted and/or fizzy taste.

### Step 7 : primary packaging.

Tablets are individually packaged into an aluminium / PE complex. Each strip contains 4 tablets.

### Example 9 - comparative studies

We performed comparative studies of the tablets of the invention, the simple synthetic mix of NAC and its excipients without tableting and the tablets produced in laboratory according to example 2 of WO2010/090611 (corresponding to the product sold under the trademark Mucoplus 1200).

The formulation of the tablets is given in table 1 below.

**Table 1 Formulation of the tablets**

| Ingredient | Mucoplus 1200 (M+1200) (mg) | tablets of the invention NAC1200 (mg) |
|---|---|---|
| NAC | 1200 | 1200 |
| NAHCO3 | 770 | 1313 |
| Citric acid | 1035 | 1087 |
| Lemon flavour | 20 | 200 |
| Aspartame | 30 | 20 |
| NaCl | 60 | 0 |
| PEG6000 | 0 | 30 |

The tests pH/solubility/effervescence were performed on normal drinking water, that is the solvent actually used by the end user. The reference volume is 150 mL, that is the volume of a normal glass of water used during meals. When appropriate, 1 tablet was dissolved in 150 ml of normal drinking water, with a short manual agitation at the end.

The results are given in table 2 below:

**Table 2 - results**

| | NAC1200 | NAC 1200 SM | Tablets example 2 WO2010/090611 |
|---|---|---|---|
| Effervescence / dissolution | Regular, not vigorous effervescence with very small boubbles that ends in 2-3' with no need of agitation. Clear solution, with some residues as supernatant visually similar to M+1200. Residues could belong to the two salts. | Rapid and vigorous: 2'-3' with small bubbles. After a first phase of remarkable effervescence due to production of CO₂, it is obtained a solution slightly opalescent but globally clear, without flocculus or deposit. | Regular and vigorous effervescence with small bubbles. The tablet disintegrates progressively flocculating fragments of material that deposit on the walls of the beker. On the surface of the liquid it remains a pellicular coating that incorporates air, maybe due to PEG. It is salted and oily. |
| | | | Disintegration and dissolution are complete in 2-3 minutes. |
| | | | The solution is opalescent and flocculus of material remain in suspension. |
| pH | 4.35 | 4.46 | 3.46 |
| Odour/taste | Lemon flavour smell and taste. The lemon flavour is not market, it is rather an equilibrium between sweet and salty. No sulfuric taste. | Lemon flavour odour. Salted and fizzy taste, with final aftertaste. Lemon flavour cannot be tasted. | Slight lemon flavour odour. Mainly salted taste, with very slight lemon flavour taste, slight final aftertaste |

### Example 10 - comparative studies

We performed comparative studies of:
1. Fluimucil 600 Zambon effervescent tablets (FMC600Z)
2. Mucoplus 1200 Turkish effervescent tablets (M+1200) bought from the turkish market
3. Effervescent tablets NAC 1200 mg according to the invention (NAC1200)
4. Laboratory mix (FMC1200SM) of NAC active principe ingredient and excipients in the same ratio than FMC600Z but with double quantity.

The formulation of Mucoplus 1200 and NAC1200 is given in table 1 above. The formulation of the FMC600Z and FMC1200SM is given in table 3 below.

**Table 3 - Formulation**

| Ingredient | FMC600Z (mg) | FMC1200SM (mg) |
|---|---|---|
| | | |
| NAC | 600 | 1200 |
| NAHCO3 | 573 | 1146 |
| Citric acid | 680 | 1360 |
| Lemon flavour | 200 | 400 |
| Aspartame | 20 | 40 |

The tests of pH/solubility/effervescence were carried out in water HPLC grade and in drinking water (the solvent actually used by the patient), in order to verify eventual variations due to the chemico-physical quality of the solvent. The reference volume was 150 mL, that is the volume of a glass of water commonly used during meals.

The characteristics of the tablets/laboratory mix are given in table 4 below

**Table 4 - characteristics**

| Characteristic | FMC600Z | M+1200 | NAC1200 | FMC1200SM |
|---|---|---|---|---|
| Appearance | intact, white | intact, white | intact, white intact, white | White powder |
| Smell | Mainly sulfuric, masse by the lemon flavour | Lemon flavour | Lemon flavour | Lemon flavour |

The dissolution of the samples in HPLC grade water / pH are given in table 5 below

**Table 5 - dissolution in HPLC grade water**

| Characteristic | FMC600Z | M+1200 | NAC1200 | FMC1200SM |
|---|---|---|---|---|
| Effervescenve / dissolution | Rapid: 1-2'. Clear solution, with big boubbles. | Rapid: 1-2'. Vigorous effervescence. Clear solution, with many small boubbles. Residues of excipients remain as supernatant and on the walls of the becker | Regular, not vigorous effervescence with very small boubbles that ends in 2-3' with no need of agitation. Clear solution, with some residues as supernatant visually similar to M+1200. Residues could belong to the two salts. | Vigorous effervescence, but slower than tablets. Dissolution is complete after 5'. Not clear, opalescent solution, but stable and homogenous. No residues of excipients. |
| pH | 3.82 | 3.42 | 4.28 | 3.83 |
| Smell/taste | Sulfuric smell. Marked lemon taste. | Marked lemon taste. The taste is salty and the lemon flavour can be noticed. | Lemon flavour smell and taste. The lemon flavour is not market, it is rather an equilibrium between sweet and salty. No sulfuric taste. | Marked lemon flavour smell and taste. |

The dissolution in drinking water / pH is given in table 6 below:

**Table 6 - pH in drinking water**

| Characteristic | FMC600Z | M+1200 | NAC1200 | FMC1200SM |
|---|---|---|---|---|
| pH | 3.88 | 3.50 | 4.35 | 3.87 |

The only difference compared to the HPLC grade water is the pH values, slightly higher because of dissolved salts of drinking water.

Tablets M+1200 and NAC1200 are equivalent in terms of apperance of the final solution. As far as taste is concerned, the salty M+1200 is a bit inconvenient, while NAC1200 taste is very gentle.

Both formulation give a complete dissolution, even if the final pH values are different compared to the reference product (FMC600Z or analogous lab mix 2X). In the same conditions of solubilization, higher pH is preferrable in term of taste. The higher pH compared to the reference (FMC600Z or lab mix 2X) is due to the higher quantity of NaHCO3 and lower quantity of citric acid.

## Claims

1. A pharmaceutical composition in the form of an effervescent tablet comprising:
(a) 1200 mg of N-acetylcysteine; and
(b) at least 1200 mg of sodium hydrogen carbonate; and
(c) less than 1200 mg of anhydrous citric acid; and
(d) at least one lubricant and/or glidant agent; and
(e) at least one sweetener and/or flavouring agent; and
said pharmaceutical composition having no sulphur smell and taste as well as a pH in the range of 4.1 and 4.4 after tablet dissolution, said composition being **characterized in that** it does not comprise sodium chloride.

2. A pharmaceutical composition according to claim 1, wherein the amount of sodium hydrogen carbonate is between 1250 mg and 1350 mg, preferably equal to 1313 mg.

3. A pharmaceutical composition according to claim 1 or 2, wherein the amount of anhydrous citric acid is between 1050 mg and 1100 mg, preferably equal to 1087 mg.

4. A pharmaceutical composition according to claims 1 to 3, wherein the lubricant is selected from the group consisting of magnesium stearate, sodium stearyl fumarate, sodium benzoate, polyethylene glycol with molecular weights of 6000 and above and colloidal silicon dioxide.

5. A pharmaceutical composition according to claim 4, wherein the lubricant is polyethylene glycol 6000.

6. A pharmaceutical composition according to claims 1 to 5, wherein sweeteners are selected from the group consisting of sucralose, sucrose, fructose, glucose, galactose, xylose, dextrose, levulose, lactose, maltose, maltodextrin, mannitol, maltitol, maltol, sorbitol, xylitol, erythritol, lactitol, isomalt, corn syrup, saccharin, saccharin salts, acesulfame potassium, aspartame, D-tryptophan, monoammonium glycyrrhizinate, neohesperidin, dihydrochalcone, thaumatin, nicotame, alitame, stevioside and cyclamate.

7. A pharmaceutical composition according to claim 6, wherein the sweetener is aspartame.

8. A pharmaceutical composition according to claims 1 to 7, wherein flavouring agents are selected from the group consisting of natural aroma oils (e.g. peppermint oil, Partridge currant oil, clove bud oil, parsley oil, eucalyptus oil, lemon oil, orange oil, etc.), menthol, mint, anethole, methyl salicylate, eucalyptol, cinnamon, 1-methylacetate, salvia, eugenol, oxanone, alpha-ionone, marjoram, lemon, orange, propenyl guaethol, cinnamon, vanilla, thymol, linalool, cinnamaldehyde, glycerol acetal, N-substituted p-menthane- 3-carboxyamide and 3,1- methoxy propane 1,2-diol.

9. A pharmaceutical composition according to claim 8, wherein the flavouring agent is lemon oil.

10. A process for preparing a pharmaceutical composition according to claims 1 to 9, which comprises
- (1) the wet granulation of N-acetylcysteine with anhydrous citric acid and optionally sodium hydrogen carbonate, then
- (2) the mixing of the granulate obtained after step (1) with the remaining excipients and
- (3) the compression of the tablet from the formulation obtained after step (1).

11. The pharmaceutical composition according to claims 1 to 9 for its use in the treatment of abnormal, viscid, or inspissated mucous secretions; in particular for secretions resulting of: chronic respiratory disorders, acute bronchopulmonary disease, tracheostomy care, during anesthesia, atelectasis due to mucous obstruction, and diagnostic bronchial studies.

12. The pharmaceutical composition according to claims 1 to 9 for its use in the treatment of hepatic injury after ingestion of hepatotoxic quantity of acetaminophen.

13. The pharmaceutical composition according to claims 1 to 9 for its use in the long term treatment of HIV with AZT.

14. The pharmaceutical composition according to claims 1 to 9 for its use in the prevention of oxidant damages and/or in the protection against cytotoxic agents such as ifosfamide and cyclophosphamide.

## Patentansprüche

1. Pharmazeutische Zusammensetzung in Form einer Brausetablette, umfassend:
(a) 1200 mg N-Acetylcystein; und
(b) mindestens 1200 mg Natriumhydrogencarbonat; und
(c) weniger als 1200 mg wasserfreie Citronensäure; und
(d) mindestens ein Gleitmittel und/oder Fließregulierungsmittel; und
(e) mindestens ein Süßungsmittel und/oder einen Geschmacksstoff;
wobei die pharmazeutische Zusammensetzung keinen Schwefelgeruch und -geschmack besitzt sowie einen pH-Wert im Bereich von 4,1 und 4,4 nach Tablettenauflösung, wobei die Zusammensetzung **dadurch gekennzeichnet ist, dass** sie kein Natriumchlorid umfasst.

2. Pharmazeutische Zusammensetzung nach Anspruch 1, wobei die Menge an Natriumhydrogencarbonat zwischen 1250 mg und 1350 mg beträgt, vorzugsweise gleich 1313 mg.

3. Pharmazeutische Zusammensetzung nach Anspruch 1 oder 2, wobei die Menge an wasserfreier Citronensäure zwischen 1050 mg und 1100 mg beträgt, vorzugsweise gleich 1087 mg.

4. Pharmazeutische Zusammensetzung nach einem der Ansprüche 1 bis 3, wobei das Gleitmittel ausgewählt ist aus der Gruppe, bestehend aus Magnesiumstearat, Natriumstearylfumarat, Natriumbenzoat, Polyethylenglykol mit Molekulargewichten von 6000 und darüber und kolloidalem Siliciumdioxid.

5. Pharmazeutische Zusammensetzung nach Anspruch 4, wobei das Gleitmittel Polyethylenglykol 6000 ist.

6. Pharmazeutische Zusammensetzung nach einem der Ansprüche 1 bis 5, wobei das Süßungsmittel ausgewählt ist aus der Gruppe, bestehend aus Sucralose, Saccharose, Fructose, Glucose, Galactose, Xylose, Dextrose, Levulose, Lactose, Maltose, Maltodextrin, Mannit, Maltit, Maltol, Sorbitol, Xylitol, Erythrit, Lactit, Isomalt, Maissirup, Saccharin, Saccharin-Salzen, Acesulfam-Kalium, Aspartam, D-Tryptophan, Monoammoniumglycyrrhizinat, Neohesperidin, Dihydrochalcon, Thaumatin, Nicotam, Alitam, Steviosid und Cyclamat.

7. Pharmazeutische Zusammensetzung nach Anspruch 6, wobei das Süßungsmittel Aspartam ist.

8. Pharmazeutische Zusammensetzung nach einem der Ansprüche 1 bis 7, wobei die Geschmacksstoffe ausgewählt sind aus der Gruppe, bestehend aus natürlichen Aromaölen (zum Beispiel Pfefferminzöl, Partridge-Johannisbeeröl, Nelkenknospenöl, Petersilienöl, Eukalyptusöl, Zitronenöl, Orangenöl, usw.), Menthol, Minze, Anethol, Methylsalicylat, Eucalyptol, Zimt, 1-Methylacetat, Salvia, Eugenol, Oxanon, alpha-Ionon, Majoran, Zitrone, Orange, Propenylguaethol, Zimt, Vanille, Thymol, Linalool, Zimtaldehyd, Glycerinacetal, N-substituiertes p-Menthan-3-carboxyamid und 3,1- Methoxypropan-1,2-diol.

9. Pharmazeutische Zusammensetzung nach Anspruch 8, wobei der Geschmacksstoff Zitronenöl ist.

10. Verfahren zur Herstellung einer pharmazeutischen Zusammensetzung nach einem der Ansprüche 1 bis 9, das umfasst:
- (1) die Nassgranulierung von N-Acetylcystein mit wasserfreier Citronensäure und wahlweise Natriumhydrogencarbonat, dann
- (2) Mischen des Granulats, das in Schritt (1) erhalten wurde, mit den verbleibenden Hilfsstoffen und
- (3) Pressen der Tablette aus der Formulierung, die nach Schritt (1) erhalten wurde.

11. Pharmazeutische Zusammensetzung nach einem der Ansprüche 1 bis 9 für ihre Verwendung bei der Behandlung von abnormalen, zähflüssigen oder eingedickten Schleimsekreten; insbesondere bei Sekreten, die aus Folgendem resultieren: chronischen Erkrankungen der Atemwege, akuten bronchopulmonalen Erkrankungen, Tracheotomiepflege während der Narkose, Atelektase aufgrund von Schleimhautobstruktion, und diagnostischen Bronchialstudien.

12. Pharmazeutische Zusammensetzung nach einem der Ansprüche 1 bis 9 für ihre Verwendung bei der Behandlung von Leberschädigung nach Einnahme einer hepatotoxischen Menge von Acetaminophen.

13. Pharmazeutische Zusammensetzung nach einem der Ansprüche 1 bis 9 für ihre Verwendung bei der Langzeitbehandlung von HIV mit AZT.

14. Pharmazeutische Zusammensetzung nach einem der Ansprüche 1 bis 9 für ihre Verwendung bei der Verhinderung von Oxidationsschäden und/oder zum Schutz vor cytotoxischen Mitteln wie Ifosfamid und Cyclophosphamid.

## Revendications

1. Composition pharmaceutique sous la forme d'un comprimé effervescent comprenant :
(a) 1200 mg de N-acétylcystéine ; et
(b) au moins 1200 mg d'hydrogénocarbonate de sodium ; et
(c) moins de 1200 mg d'acide citrique anhydre ; et
(d) au moins un lubrifiant et/ou un agent de glissement ; et
(e) au moins un édulcorant et/ou un agent aromatisant ; et
ladite composition pharmaceutique n'ayant ni l'odeur ni le goût de soufre et ayant un pH situé dans la plage de 4,1 et 4,4 après la dissolution du comprimé, ladite composition étant **caractérisée en ce qu'**elle ne comprend pas de chlorure de sodium.

2. Composition pharmaceutique selon la revendication 1, dans laquelle la quantité d'hydrogénocarbonate de sodium se situe entre 1250 mg et 1350 mg, de préférence est égale à 1313 mg.

3. Composition pharmaceutique selon la revendication 1 ou 2, dans laquelle la quantité d'acide citrique anhydre se situe entre 1050 mg et 1100 mg, de préférence est égale à 1087 mg.

4. Composition pharmaceutique selon les revendications 1 à 3, dans laquelle le lubrifiant est sélectionné dans le groupe consistant en stéarate de magnésium, fumarate de stéaryle sodique, benzoate de sodium, polyéthylène glycol avec des poids moléculaires de 6000 et supérieurs et dioxyde de silicium colloïdal.

5. Composition pharmaceutique selon la revendication 4, dans laquelle le lubrifiant est le polyéthylène glycol 6000.

6. Composition pharmaceutique selon les revendications 1 à 5, dans laquelle les édulcorants sont sélectionnés dans le groupe consistant en sucralose, saccharose, fructose, glucose, galactose, xylose, dextrose, lévulose, lactose, maltose, maltodextrine, mannitol, maltitol, maltol, sorbitol, xylitol, érythritol, lactitol, isomalt, sirop de maïs, saccharine, sels de saccharine, acésulfame potassium, aspartame, D-tryptophane, monoglycyrrhizinate d'ammonium, néohespéridine, dihydrochalcone, thaumatine, nicotame, alitame, stévioside et cyclamate.

7. Composition pharmaceutique selon la revendication 6, dans laquelle l'édulcorant est l'aspartame.

8. Composition pharmaceutique selon les revendications 1 à 7, dans laquelle les agents aromatisants sont sélectionnés dans le groupe consistant en essences aromatiques naturelles (par exemple, essence de menthe poivrée, essence de baie de perdrix, essence de girofle, essence de persil, essence d'eucalyptus, essence de citron, essence d'orange, etc.), menthol, menthe, anéthol, salicylate de méthyle, eucalyptol, cannelle, 1-méthylacétate, sauge, eugénol, oxanone, alpha-ionone, marjolaine, citron, orange, propényl-guaéthol, cannelle, vanille, thymol, linalool, cinnamaldéhyde, acétal de glycérol, p-menthane-3-carboxyamide N-substitué et 3,1-méthoxy-propane-1,2-diol.

9. Composition pharmaceutique selon la revendication 8, dans laquelle l'agent aromatisant est l'essence de citron.

10. Procédé de préparation d'une composition pharmaceutique selon les revendications 1 à 9, qui comprend
- (1) la granulation humide de N-acétylcystéine avec de l'acide citrique anhydre et éventuellement de l'hydrogénocarbonate de sodium, puis
- (2) le mélange du granulat obtenu après l'étape (1) avec le reste des excipients et
- (3) la compression du comprimé à partir de la formulation obtenue après l'étape (1).

11. Composition pharmaceutique selon les revendications 1 à 9 pour son utilisation dans le traitement de sécrétions muqueuses anormales, visqueuses, ou épaissies ; en particulier pour des sécrétions résultant de : troubles respiratoires chroniques, maladies broncho-pulmonaires aiguës, soins de trachéotomie, durant une anesthésie, une atélectasie due à une obstruction muqueuse, et études bronchiques de diagnostic.

12. Composition pharmaceutique selon les revendications 1 à 9, pour son utilisation dans le traitement de lésions hépatiques après ingestion d'une quantité hépatotoxique de paracétamol.

13. Composition pharmaceutique selon les revendications 1 à 9, pour son utilisation dans le traitement à long terme du VIH avec de l'AZT.

14. Composition pharmaceutique selon les revendications 1 à 9, pour son utilisation dans la prévention des lésions oxydatives et/ou dans la protection contre des agents cytotoxiques tels que l'ifosfamide et le cyclophosphamide.
